# EUROPÄISCHE PATENTANMELDUNG

(11) **EP 1 688 049 A1**
(43) Veröffentlichungstag der Anmeldung: **09.08.2006**
(21) Anmeldenummer: 05002212.8
(22) Anmeldetag: 03.02.2005
(51) Int. Cl.: A23L 1/30, A23L 1/187, A23D 7/00, A61P 1/18, A61K 31/20

(54) **Diätetisches Lebensmittel**

(71) Anmelder: findusFit Sportdiätetika GmbH, 94344 Wiesenfelden (DE)
(72) Erfinder: Sirtl, Thomas, 94344 Wiesenfelden (DE)
(74) Vertreter: Mühlbauer, Robert

(57) **Zusammenfassung**

Die vorliegende Erfindung betrifft ein diätetisches Lebensmittel, insbesondere in Form von Diätgetränken, Desserts, Riegeln, Suppen, Soßen, Gebäck, wobei die enthaltenen Fettsäuren im wesentlichen natürliche Fettsäuren mit einer Kettenlänge bis C 18 sind und im wesentlichen als freie Fettsäuren oder deren Salze und gebunden als Monoglyceride vorliegen, und wobei die freien und als Monoglyceride gebundenen Fettsäuren mindestens teilweise gesättigte und/oder einfach ungesättigte Fettsäuren sind.

Die vorliegende Erfindung betrifft ferner eine Fettsäuremischung, insbesondere zur Herstellung von diätetischen Lebensmitteln, wobei die enthaltenen Fettsäuren im wesentlichen natürliche Fettsäuren mit einer Kettenlänge bis C 18 sind und im wesentlichen als freie Fettsäuren oder deren Salze und gebunden als Monoglyceride vorliegen, und wobei die freien und als Monoglyceride gebundenen Fettsäuren mindestens teilweise gesättigte und/oder einfach ungesättigte Fettsäuren sind.

Die vorliegende Erfindung betrifft ferner die Verwendung einer erfindungsgemäßen Fettsäuremischung.

Die vorliegende Erfindung betrifft ferner ein Lebensmittel, dem eine erfindungsgemäße Fettsäuremischung zugemischt ist.

Die vorliegende Erfindung betrifft ferner die Verwendung eines erfindungsgemä-βen Lebensmittels zur Ernährung von Patienten mit ernährungsbedingten Krankheiten sowie Erkrankungen, die auf einer Störung der Aufnahme, des Abbaus oder der Ausscheidung bestimmter Nahrungsbestandteile oder ihrer Folgeprodukte beruhen.

Die vorliegende Erfindung betrifft ferner ein Verfahren zur Herstellung einer erfindungsgemäßen Fettsäuremischung gekennzeichnet durch folgende Arbeitsschritte:
- Einwiegen mindestens einer Fettsäure, Glycerinmonofettsäureester und einem Fließregulierungsmittel in einen geeigneten Mixer, wobei die Fettsäure mit einem Anteil von ca. 66 % und der Glycerinmonofettsäureester mit einem Anteil von ca. 33 % und das Fließregulierungsmittel mit einem Anteil von ca. 1 % eingewogen werden;
- Mischen des Gemisches bis zur Homogenität;
- Abfüllen in geeignete Behältnisse.

## Beschreibung

Die vorliegende Erfindung betrifft ein diätetisches Lebensmittel, insbesondere in Form von Pulvern, Diätgetränken, Desserts, Riegeln, Suppen, Soßen, Gebäck, wobei die enthaltenen Fettsäuren im Wesentlichen natürliche Fettsäuren mit einer Kettenlänge C 18 sind und im Wesentlichen als freie Fettsäuren oder deren Salze und gebunden als Monoglyceride vorliegen.

Die erfindungsgemäßen diätetischen Lebensmittel sind in erster Linie zur Ernährung von Patienten mit ernährungsbedingten Krankheiten sowie Erkrankungen, die auf eine Störung der Aufnahme, des Abbaus oder der Ausscheidung bestimmter Nahrungsbestandteile oder ihrer Folgeprodukte beruhen, gedacht. Besonders geeignet sind die erfindungsgemäßen diätetischen Lebensmittel zur Ernährung von Patienten, die unter einer Pankreasinsuffizienz leiden. Pankreasinsuffizienz macht sich in erster Linie durch Malnutrition bemerkbar. Hierunter versteht man eine Fehl- bzw. Mangelernährung, d. h. jede Form der Nahrungszufuhr, bei der die dem Körper zugeführte Menge an Energie oder an einem oder mehreren Nährstoffen für längere Zeit eine negative Bilanz aufweist. Dabei kommt es zu Gewichtsverlust und zur Unterversorgung mit Nährstoffen.

Pankreasinsuffizienz resultiert aus einer verminderten Ausschüttung von Verdauungsenzymen (z.B. Amylasen, Lipasen, Trypsin) was wiederum zu Fettstühlen, Übelkeit, Erbrechen, Gewichtsabnahme führt. Eine Pankreasinsuffizienz kann erworben sein, z. B. chronische Pankreatitis, endokrine Pankreatitis oder Pankreastumoren. Sie kann auch angeboren sein. Häufigstes Beispiel ist die Mukoviszidose.

Die Folgen der Mangelernährung bedingt durch die Pankreasinsuffizienz bei Mukoviszidose sind am bekanntesten und auch am fundiertesten beschrieben. Bedingt durch den Mangel an Pankreasenzymen kommt es zu einer Unterversorgung im Energiebereich, zu Minderwachstum, zu verringerter Muskelmasse und einer verkürzten Lebenserwartung. Vor allem durch den Mangel an Lipasen wird Fett nur ungenügend aufgespalten und über 7 % des Fettanteils der Nahrung wird unverdaut ausgeschieden (Fettstuhl). Bei einem Drittel der Patienten gehen über 20 % des Fettanteils der Nahrung, trotz Enzymsubstitution, verloren. Der Fettstuhl bewirkt nicht nur eine verminderte Energiezufuhr, sondern auch fäkale Stickstoffverluste bis zu 50 %, also eine verminderte Proteinaufnahme. Diese verminderte Proteinaufnahme bewirkt eine Verringerung der Muskelmasse und bei Heranwachsenden ein verringertes Wachstum. Im Fettstuhl sind die fettlöslichen Vitamine (A, D, E, K) gelöst und können vom Körper oft nur ungenügend aufgenommen werden. In der Regel ist auch die Stärkedigestion beeinträchtigt. Dies bedeutet eine verminderte Energiezufuhr auch durch Kohlenhydratmangel.

Hochkalorische Diäten mit hohen Fettanteilen und Pankreasenzymsubstituion konnten nur teilweise Erfolge erzielen (vgl. Sybille Koletzko u. a. "Akutelle Aspekte der Ernährungstherapie bei zystischer Fibrose" in Monatsschrift Kinderheilkunde, 1994, 142; 432-445). Auch hier gehen bis zu 20 % Fett unverdaut verloren. Dies bewirkt wiederum die bereits oben genannten Verluste auch bei weiteren Nährstoffen.

Aufgrund der oben gemachten Ausführungen wurden in der Vergangenheit Versuche unternommen, diätetische Lebensmittel bereit zu stellen, die von Patienten mit Pankreasinsuffizenz ― insbesondere hinsichtlich der Fettverwertung ― besser verwertet werden können.
Fette bestehen aus Fettsäuretriglyceriden, also aus natürlichen Fettsäuren verschiedener Kettenlängen, die mit Glyzerin zu Triglyceriden verestert sind. Bei der Verdauung spaltet das Pankreasenzym Lipase Fette in Fettsäuren und Glyzerin, teilweise auch in Fettsäuren und Monoglyceride. Die nun vorliegenden freien Fettsäuren werden von der Darmschleimhaut im Dünndarm resorbiert, wobei nicht gespaltene Fette nicht resorbiert werden können (Entstehung von Fettstuhl). In der Darmwand erfolgt nun die Synthese von Fettpartikeln, sogenannten Chylomikronen, unter Veresterung von Fettsäuren und Glyzerin. Dabei werden die fettlöslichen Vitamine mit eingeschlossen. Die Chylomikronen werden vom Blut aufgenommen und den einzelnen Organen zur Verfügung gestellt. In den Organen werden die Fettmolekühle durch Lipasen, die unabhängig von den Pankreaslipasen produziert werden, erneut gespalten und die Fettsäuren stehen als Hauptenergielieferanten oder als Bausteine zur Verfügung.

Um eine verbesserte Fettverwertung ― insbesondere von Patienten mit Pankreasinsuffizienz - zu ermöglichen, werden in DE 102 21 754 A1 Lebensmittel beschrieben, bei denen die Fette ganz oder teilweise durch freie, natürlich vorkommende Fettsäuren ersetzt wurden. Dies hat den Vorteil, dass die Fettsäuren sofort (ohne vorherige Abspaltung aus Triglyceriden) in einer physiologisch verwertbaren Form zur Verfügung stehen. Eine enzymatische Spaltung durch Pankreaslipasen ist also nicht mehr nötig. Die Darmschleimhaut kann die Fettsäuren nach der Magenpassage sofort aufnehmen und Chylomikronen bilden.

Ein Nachteil der in DE 102 21 754 A1 genannten Lebensmitteln besteht darin, dass die freien Fettsäuren zwar gut vom Dünndarm resorbierbar sind, die Synthese von Chylomikronen in der Darmwand jedoch nicht optimal von statten geht. Zudem ist die Verteilung der freien Fettsäuren im Dünndarm nicht optimal, wodurch auch die Verfügbarkeit der freien Fettsäuren verbesserungsfähig ist.

In DE 197 57 414 A1 wird ein diätetisches Nahrungsmittel beschrieben, welches eine Fettmischung auf Basis von Ölen, Fetten und/oder Lecithinen mit mehrfach ungesättigten Fettsäuren enthält. Die Fettmischung kann dabei auch ausschließlich aus freien Fettsäuren bestehen, wobei es sich bei den freien Fettsäuren in erster Linie um essentielle Fettsäuren, wie z. B. Linolsäure oder Alpha-Linolensäure, handelt.
Ein gravierender Nachteil der in dieser Druckschrift genannten Lebensmittel besteht darin, dass die darin enthaltenen ungesättigten Fettsäuren einen relativ geringen Energiegehalt aufweisen, was z.B. für Patienten mit Pankreasinsuffizienz von Nachteil ist. Zudem verursachen sie einen unangenehmen Geschmack des Lebensmittels.

In DE 28 448 61 A1 wird ein diätetisches Lebensmittel beschrieben, welches dadurch hergestellt wird, dass der festen oder flüssigen Nahrung freie Fettsäuren und Monoglyceride oder ihre Mischungen mit Diglyceriden, Fetten und/oder mittelkettigen Triglyceriden zugemischt werden. Die freien Fettsäuren werden aus üblichen Speiseölen und Speisefetten durch chemische und/oder enzymatische Reaktionen gewonnen. Dies bedeutet, dass bei der Herstellung dieser Lebensmittel undefinierte Gemische aus verschiedensten freien Fettsäuren, insbesondere aus mehrfach ungesättigten Fettsäuren, verwendet werden. Die in dieser Druckschrift genannten Lebensmittel haben dadurch wiederum den Nachteil, dass die darin enthaltenen mehrfach ungesättigten Fettsäuren einen relativ geringen Energiegehalt aufweisen und zudem einen unangenehmen Geschmack der Lebensmittel verursachen.

Der Erfindung liegt die Aufgabe zugrunde, ein diätetisches Lebensmittel zur Verfügung zu stellen, das die Nachteile des Standes der Technik überwindet, insbesondere hinsichtlich der Verwertbarkeit verbessert ist.

Diese Aufgabe wird erfindungsgemäß durch ein diätetisches Lebensmittel, insbesondere in Form von Diätgetränken, Desserts, Riegeln, Suppen, Soßen, Gebäck, wobei die enthaltenen Fettsäuren im Wesentlichen natürliche Fettsäuren mit einer Kettenlänge bis C 18 sind und im Wesentlichen als freie Fettsäuren oder deren Salze und gebunden als Monoglyceride vorliegen, und wobei die freien und als Monoglyceride gebundenen Fettsäuren mindestens teilweise gesättigte und/oder einfach ungesättigte Fettsäuren sind.

Das erfindungsgemäße Lebensmittel hat sich überraschenderweise als besonders gut verwertbar - insbesondere für Patienten mit Pankreasinsuffizienz - herausgestellt.

Durch die Tatsache, dass die Fettsäuren im Wesentlichen zum Teil als freie Fettsäuren (oder deren Salze) und zum Teil gebunden als Monoglyceride vorliegen, wird eine wesentliche Verbesserung der Verteilung der Fettsäuren im Darm gewährleistet, da die Monoglyceride auch als Emulgator für die freien Fettsäuren fungieren können. Dies bewirkt wiederum eine wesentliche Verbesserung der Verfügbarkeit der freien Fettsäuren.
Ein weiterer Vorteil der Monoglyceride besteht darin, dass diese - wie die freien Fettsäuren - von der Darmschleimhaut im Dünndarm resorbiert werden. Dadurch stehen in der Darmwand die beiden zur Synthese der Chylomikronen nötigen Bausteine (freie Fettsäuren und Glyzerin-Baustein) zur Verfügung.
Dadurch, dass die Monoglyceride auch darmgängig sind, leisten diese zudem einen nicht zu vernachlässigenden energetischen Beitrag.

Aufgrund der Tatsache, dass die freien und als Monoglyceride gebundenen Fettsäuren des erfindungsgemäß diätetischen Lebensmittels mindestens teilweise gesättigte und/oder einfach ungesättigte Fettsäuren sind, weist das erfindungsgemäße Lebensmittel einen besonders hohen Energiegehalt auf. Dies ist besonders für Patienten mit Pankreasinsuffizienz von Vorteil. Zudem weist das erfindungsgemäße Lebensmittel einen wesentlich verbesserten Geschmack auf im Vergleich zu den diätetischen Lebensmitteln aus dem Stand der Technik, die in erster Linie mehrfach ungesättigte Fettsäuren enthalten.

Bei einer besonders bevorzugten Ausführungsform des erfindungsgemäßen diätetischen Lebensmittels beträgt das Molverhältnis der freien Fettsäuren zu den Monoglyceriden ca. 2 : 1. Dieses Molverhältnis ist das optimale für die Synthese von Triglyceriden aus den vorhandenen Monoglyceriden und den Fettsäuren zur Bildung von Chylomikronen.

Vorzugsweise sind die freien und als Monoglyceride gebundenen Fettsäuren im Wesentlichen, insbesondere ausschließlich, gesättigte und/oder einfach ungesättigte Fettsäuren. Durch das ausschließliche Vorhandensein von gesättigten und/oder einfach ungesättigten Fettsäuren in dem erfindungsgemäßen diätetischen Lebensmittel weist dieses einen besonders hohen Energiegehalt auf. Dies ist ― wie bereits oben erwähnt - beispielsweise vorteilhaft für Patienten, die unter Pankreasinsuffizienz leiden, da diese Patienten in der Regel unter starkem Gewichtsverlust aufgrund erniedrigter Energiebereitstellung leiden.

Mit Vorteil sind die freien und als Monoglyceride gebundenen Fettsäuren im Wesentlichen Palmitinsäure und/oder Stearinsäure und/oder Ölsäure. Diese Säuren sind besonders energiereich und haben sich hinsichtlich der Verwertbarkeit und des Geschmacks als besonders vorteilhaft erwiesen.

Neben gesättigten und/oder einfach ungesättigten Fettsäuren können mehrfach ungesättigte Fettsäuren, insbesondere Linolsäure und/oder α-Linolensäure enthalten sein. Aus diesen Säuren können praktisch alle sonstigen vom Körper benötigten Fettsäuren, z. B. Arachidonsäure, vom Körper synthetisiert werden.

Die vorliegende Erfindung betrifft ferner eine Fettsäuremischung, insbesondere zur Herstellung von diätetischen Lebensmitteln, wobei die enthaltenen Fettsäuren im Wesentlichen natürliche Fettsäuren mit einer Kettenlänger bis C 18 sind und im Wesentlichen als freie Fettsäuren oder deren Salze und gebunden als Monoglyceride vorliegen und wobei die freien und als Monoglyceride gebundenen Fettsäuren mindestens teilweise gesättigte und/oder einfach ungesättigte Fettsäuren sind.

Die erfindungsgemäße Fettsäuremischung eignet sich insbesondere zur Herstellung eines erfindungsgemäßen diätetischen Lebensmittels.

Besonders bevorzugt beträgt das Molverhältnis der freien Fettsäuren zu den Monoglyceriden ca. 2 : 1. Dieses Verhältnis ist das optimale zur Synthese von Triglyceriden und damit von Chylomikronen in der Darmwand.

Vorzugsweise sind die freien und als Monoglyceride gebundenen Fettsäuren im Wesentlichen, insbesondere ausschließlich, gesättigte und/oder einfach ungesättigte Fettsäuren.

Mit Vorteil sind die freien und als Monoglyceride gebundene Fettsäuren im Wesentlichen Palmitinsäure und/oder Stearinsäure und/oder Ölsäure.

Neben gesättigten und/oder einfach ungesättigten Fettsäuren kann die erfindungsgemäße Fettsäuremischung mehrfach ungesättigte Fettsäuren, insbesondere Linolsäure und/oder Alpha-Linolensäure enthalten.

Die vorliegende Erfindung betrifft ferner die Verwendung einer erfindungsgemä-βen Fettsäuremischung zur Herstellung eines diätetischen Lebensmittels.

Die vorliegende Erfindung betrifft ferner ein Lebensmittel, welches dadurch gekennzeichnet ist, dass ihm eine erfindungsgemäße Fettsäuremischung zugemischt wurde. Hierbei kann es sich um ein vollsynthetisches Lebensmittel handeln. Es kann sich aber auch beispielsweise um ein handelsübliches Lebensmittel handeln (beispielsweise Suppenpulver, Fertiggerichte, Puddingpulver usw.), dem eine erfindungsgemäße Fettsäuremischung zugemischt wurde. Solche handelsüblichen Lebensmittel, die mit einer erfindungsgemäßen Fettsäuremischung versetzt wurden, können bereits vor dem Zumischen der erfindungsgemäßen Fettsäuremischung einen Eigenfettanteil aufweisen.

Die vorliegende Erfindung betrifft ferner die Verwendung eines erfindungsgemäßen Lebensmittels oder einer erfindungsgemäßen Fettsäuremischung zur Ernährung von Patienten mit ernährungsbedingten Krankheiten sowie Erkrankungen, die auf einer Störung der Aufnahme, des Abbaus oder der Ausscheidung bestimmter Nahrungsbestandteile oder ihrer Folgeprodukte beruhen.

Bei einer bevorzugten Verwendung sind die Erkrankungen mit einer Pankreasinsuffizienz verbunden.

Die vorliegende Erfindung betrifft ferner ein Verfahren zur Herstellung einer erfindungsgemäßen Fettsäuremischung gekennzeichnet durch folgende Arbeitsschritte:
- Einwiegen mindestens einer Fettsäure, Glycerinmonofettsäureester und einem Fließregulierungsmittel in einen geeigneten Mixer, wobei die Fettsäure mit einem Anteil von ca. 66 % und der Glycerinmonofettsäureester mit einem Anteil von ca. 33 % und das Fließregulierungsmittel mit einem Anteil von ca. 1 % eingewogen werden;
- Mischen des Gemisches bis zur Homogenität;
- Abfüllen des Gemisches in geeignete Behältnisse.

Vorzugsweise werden bei dem erfindungsgemäßen Verfahren zwei Fettsäuren, vorzugsweise Stearinsäure und Palmitinsäure, mit einem Anteil von jeweils ca. 33 % eingewogen.

Weitere Einzelheiten und Merkmale der Erfindung ergeben sich aus der nachfolgenden Beschreibung der Herstellung von erfindungsgemäßen Lebensmitteln in Verbindung mit den Unteransprüchen. Hierbei können die jeweiligen Merkmale für sich allein oder zu mehreren in Kombination miteinander verwirklicht sein.

### Beispiele zur Herstellung erfindungsgemäßer Lebensmittel:

**Neutrales Pulver zur Beimischung nach Bedarf**

| Zusammensetzung: | in % |
|---|---|
| Stearinsäure | 33 |
| Palmitinsäure | 33 |
| Glycerin-Mono-Fettsäureester | 33 |
| Fließregulierungsmittel (z.B. Maltodextrin, Aerosil) | 1 |

### Herstellung:

Einwiegen der einzelnen Bestandteile in einen geeigneten Mischer, Mischen bis zur Homogenität. Anschließend wird abgefüllt in Dosen, oder Beutel.

**Müsliriegel**

| Zusammensetzung: | in % |
|---|---|
| Stearinsäure | 7 |
| Palmitinsäure | 7 |
| Glycerin-Mono-Fettsäureester | 7 |
| Haferflocken | 35 |
| Mandelkrokant | 12 |
| Fruchtpulver | 14 |
| Glukosesirup | 14 |
| Sonnenblumenöl | 4 |

### Herstellung:

Alle Zutaten, außer dem Glukosesirup, werden gewogen und dann in einen geeigneten Mischer gegeben und homogen gemischt. Der Glukosesirup wird unter kneten langsam zugegeben. Die entstandene Masse wird zu einem Strang geformt, oder zu einem Teppich ausgewalzt. Danach geschnitten und getrocknet und einzeln in Folie verpackt.

**Schokoladenpudding: (Fertigpulver)**

| Zusammensetzung: | in % |
|---|---|
| Stearinsäure | 8 |
| Palmitinsäure | 8 |
| Glycerin-Mono-Fettsäureester | 8 |
| Stärke | 18 |
| Modifizierte Stärke | 8 |
| Kakaopulver entölt | 10 |
| Zucker | 34,95 |
| Maltodextrin | 5 |
| Aroma | 0,05 |

### Herstellung:

Alle Komponenten werden abgewogen, in einem geeigneten Mischer bis zur Homogenität gemischt. Anschließend in Beutel abgepackt.

**Diätmargarine**

| Zusammensetzung: | in % |
|---|---|
| Stearinsäure | 8 |
| Palmitinsäure | 8 |
| Glycerin-Mono-Fettsäureester | 8 |
| Pflanzliche Öle | 30 |
| Pflanzliche Fette | 20 |
| Wasser | 25,34 |
| Speisesalz | 0,3 |
| Lecithine | 0,2 |
| Zitronensäure | 0,1 |
| Aroma und Farbstoff Carotin | 0,01 |

### Herstellung

Alle Komponenten werden gewogen, in einem Mischer bei 60°C homogen gemischt und dann unter ständigem Rühren abgekühlt. Dann bei etwa 30°C in Dosen abgefüllt.

## Patentansprüche

1. Diätetisches Lebensmittel, insbesondere in Form von Pulvern, Diätgetränken, Desserts, Riegeln, Suppen, Soßen, Gebäck, wobei die enthaltenen Fettsäuren im wesentlichen natürliche Fettsäuren mit einer Kettenlänge bis C 18 sind und im wesentlichen als freie Fettsäuren oder deren Salze und gebunden als Monoglyceride vorliegen, und wobei die freien und als Monoglyceride gebundenen Fettsäuren mindestens teilweise gesättigte und/oder einfach ungesättigte Fettsäuren sind.

2. Diätetisches Lebensmittel nach Anspruch 1, **dadurch gekennzeichnet, dass** das Molverhältnis der freien Fettsäuren zu den Monoglyceriden ca. 2 : 1 beträgt.

3. Diätetisches Lebensmittel nach einem der Ansprüche 1 oder 2, **dadurch gekennzeichnet, dass** die freien und als Monoglyceride gebundenen Fettsäuren im wesentlichen, insbesondere ausschließlich, gesättigte und/oder einfach ungesättigte Fettsäuren sind.

4. Diätetisches Lebensmittel nach einem der vorhergehenden Ansprüche, **dadurch gekennzeichnet, dass** die freien und als Monoglyceride gebundenen Fettsäuren im wesentlichen Palmitinsäure und/oder Stearinsäure und/oder Ölsäure sind.

5. Diätetisches Lebensmittel nach einem der vorhergehenden Ansprüche, **dadurch gekennzeichnet, dass** neben gesättigten und/oder einfach ungesättigten Fettsäuren mehrfach ungesättigte Fettsäuren, insbesondere Linolsäure und/oder Alpha-Linolensäure, enthalten sind.

6. Fettsäuremischung, insbesondere zur Herstellung von diätetischen Lebensmitteln, wobei die enthaltenen Fettsäuren im wesentlichen natürliche Fettsäuren mit einer Kettenlänge bis C 18 sind und im wesentlichen als freie Fettsäuren oder deren Salze und gebunden als Monoglyceride vorliegen, und wobei die freien und als Monoglyceride gebundenen Fettsäuren mindestens teilweise gesättigte und/oder einfach ungesättigte Fettsäuren sind.

7. Fettsäuremischung nach Anspruch 6, **dadurch gekennzeichnet, dass** das Molverhältnis der freien Fettsäuren zu den Monoglyceriden ca. 2 : 1 beträgt.

8. Fettsäuremischung nach einem der Ansprüche 6 oder 7, **dadurch gekennzeichnet, dass** die freien und als Monoglyceride gebundenen Fettsäuren im wesentlichen, insbesondere ausschließlich, gesättigte und/oder einfach ungesättigte Fettsäuren sind.

9. Fettsäuremischung nach einem der Ansprüche 6 bis 8, **dadurch gekennzeichnet, dass** die freien und als Monoglyceride gebundenen Fettsäuren im wesentlichen Palmitinsäure und/oder Stearinsäure und/oder Ölsäure sind.

10. Fettsäuremischung nach einem der Ansprüche 6 bis 9, **dadurch gekennzeichnet, dass** neben gesättigten und/oder einfach ungesättigten Fettsäuren mehrfach ungesättigte Fettsäuren, insbesondere Linolsäure und/oder Alpha-Linolensäure, enthalten sind.

11. Verwendung einer Fettsäuremischung nach einem der Ansprüche 6 bis 9 zur Herstellung eines diätetischen Lebensmittels.

12. Lebensmittel, **dadurch gekennzeichnet, dass** ihm eine Fettsäuremischung nach einem der Ansprüche 6 bis 9 zugemischt wurde.

13. Verwendung eines Lebensmittels nach einem der Ansprüche 1 bis 5 oder 12 oder einer Fettsäuremischung nach einem der Ansprüche 6 bis 10 zur Ernährung von Patienten mit ernährungsbedingten Krankheiten sowie Erkrankungen, die auf einer Störung der Aufnahme, des Abbaus oder der Ausscheidung bestimmter Nahrungsbestandteile oder ihrer Folgeprodukte beruhen.

14. Verwendung nach Anspruch 13, **dadurch gekennzeichnet, dass** die Erkrankungen mit einer Pankreasinsuffizienz verbunden sind.

15. Verfahren zur Herstellung einer Fettsäuremischung nach einem der Ansprüche 6 bis 10 **gekennzeichnet durch** folgende Arbeitsschritte:
- Einwiegen mindestens einer Fettsäure, Glycerinmonofettsäureester und einem Fließregulierungsmittel in einen geeigneten Mixer, wobei die Fettsäure mit einem Anteil von ca. 66 % und der Glycerinmonofettsäureester mit einem Anteil von ca. 33 % und das Fließregulierungsmittel mit einem Anteil von ca. 1 % eingewogen werden;
- Mischen des Gemisches bis zur Homogenität;
- Abfüllen des Gemisches in geeignete Behältnisse.

16. Verfahren nach Anspruch 15, **dadurch gekennzeichnet, dass** zwei Fettsäuren, vorzugsweise Stearinsäure und Palmitinsäure, mit einem Anteil von jeweils 33 % eingewogen werden.

## Geänderte Patentansprüche

### Geänderte Patentansprüche gemäss Regel 86(2) EPÜ.

**1.** Diätetisches Lebensmittel, insbesondere in Form von Pulvern, Diätgetränken, Desserts, Riegeln, Suppen, Soßen, Gebäck, wobei die enthaltenen Fettsäuren im wesentlichen natürliche Fettsäuren mit einer Kettenlänge bis C 18 sind und im wesentlichen als freie Fettsäuren oder deren Salze und gebunden als Monoglyceride vorliegen, und wobei die freien und als Monoglyceride gebundenen Fettsäuren mindestens teilweise gesättigte und/oder einfach ungesättigte Fettsäuren sind.

**2.** Diätetisches Lebensmittel nach Anspruch 1, **dadurch gekennzeichnet, dass** das Molverhältnis der freien Fettsäuren zu den Monoglyceriden ca. 2 : 1 beträgt.

**3.** Diätetisches Lebensmittel nach einem der Ansprüche 1 oder 2, **dadurch gekennzeichnet, dass** die freien und als Monoglyceride gebundenen Fettsäuren im wesentlichen, insbesondere ausschließlich, gesättigte und/oder einfach ungesättigte Fettsäuren sind.

**4.** Diätetisches Lebensmittel nach einem der vorhergehenden Ansprüche, **dadurch gekennzeichnet, dass** die freien und als Monoglyceride gebundenen Fettsäuren im wesentlichen Palmitinsäure und/oder Stearinsäure und/oder Ölsäure sind.

**5.** Diätetisches Lebensmittel nach einem der vorhergehenden Ansprüche, **dadurch gekennzeichnet, dass** neben gesättigten und/oder einfach ungesättigten Fettsäuren mehrfach ungesättigte Fettsäuren, insbesondere Linolsäure und/oder Alpha-Linolensäure, enthalten sind.

**6.** Verwendung einer Fettsäuremischung, wobei die enthaltenen Fettsäuren im wesentlichen natürliche Fettsäuren mit einer Kettenlänge bis C 18 sind und im wesentlichen als freie Fettsäuren oder deren Salze und gebunden als Monoglyceride vorliegen, und wobei die freien und als Monoglyceride gebundenen Fettsäuren mindestens teilweise gesättigte und/oder einfach ungesättigte Fettsäuren sind, zur Herstellung eines diätetischen Lebensmittels.

**7.** Lebensmittel, **dadurch gekennzeichnet, dass** ihm eine Fettsäuremischung zugemischt wurde, wobei die enthaltenen Fettsäuren im wesentlichen natürliche Fettsäuren mit einer Kettenlänge bis C 18 sind und im wesentlichen als freie Fettsäuren oder deren Salze und gebunden als Monoglyceride vorliegen, und wobei die freien und als Monoglyceride gebundenen Fettsäuren mindestens teilweise gesättigte und/oder einfach ungesättigte Fettsäuren sind.

**8.** Verwendung eines Lebensmittels nach einem der Ansprüche 1 bis 5 oder 7 zur Ernährung von Patienten mit ernährungsbedingten Krankheiten sowie Erkrankungen, die auf einer Störung der Aufnahme, des Abbaus oder der Ausscheidung bestimmter Nahrungsbestandteile oder ihrer Folgeprodukte beruhen.

**9.** Verwendung nach Anspruch 8, **dadurch gekennzeichnet, dass** die Erkrankungen mit einer Pankreasinsuffizienz verbunden sind.

**10.** Verfahren zur Herstellung eines Lebensmittels nach einem der Ansprüche 1 bis 5, **gekennzeichnet durch** folgende Arbeitsschritte:
a. Einwiegen mindestens einer Fettsäure, Glycerinmonofettsäureester und einem Fließregulierungsmittel in einen geeigneten Mixer, wobei die Fettsäure mit einem Anteil von ca. 66 % und der Glycerinmonofettsäureester mit einem Anteil von ca. 33 % und das Fließregulierungsmittel mit einem Anteil von ca. 1 % eingewogen werden;
b. Mischen des Gemisches bis zur Homogenität;
c. Abfüllen des Gemisches in geeignete Behältnisse.

**11.** Verfahren nach Anspruch 15, **dadurch gekennzeichnet, dass** zwei Fettsäuren, vorzugsweise Stearinsäure und Palmitinsäure, mit einem Anteil von jeweils 33 % eingewogen werden.
